# EUROPEAN PATENT SPECIFICATION

(11) **EP 1 517 617 B1**
(45) Date of publication and mention of the grant of the patent: **17.01.2007**
(21) Application number: 02754551.6
(22) Date of filing: 02.07.2002
(51) Int. Cl.: A23G 4/00

(54) **COMPRESSED CHEWING GUM**
ZUSAMMENGEPRESSTE KAUGUMMITABLETTE
GOMME A MACHER COMPRIMEE

(43) Date of publication of application: 30.03.2005
(73) Proprietor: Gumlink A/S, 7100 Vejle (DK)
(72) Inventor: ANDERSEN, Rita, B ge, M lholm, DK-7100 Vejle (DK); BOESEN, Dorthe, Schackinger, DK-7100 Vejle (DK); SCHMIDT, Niels, Ravn, DK-7150 Barrit (DK); NISSEN, Vibeke, DK-7000 Fredericia (DK)
(74) Representative: Olesen, Kaj
(86) International application number: PCT/DK2002/000462
(87) International publication number: WO 2004/004479

(56) References cited:
- EP-A- 0 151 344
- EP-A- 0 221 850
- DE-A- 2 808 160
- US-A- 4 000 321
- US-A- 4 737 366
- US-A- 5 017 385

## Description

### Field of the invention

The present invention relates to a chewing gum manufacturing process and a gum base granulate.

### Background of the invention

Several different processes for manufacturing of chewing gum are known within the art. The different processes may be overall categorized in basically two different processes; that is chewing gum mechanically mixed on the basis of a gum base compounds or chewing gum compressed on the basis of more or less discrete gum base particles. The first type of chewing gum generally benefits of a very comfortable texture, among several different parameters, most likely due to the mechanically mixing of the polymers and for example the flavors. One disadvantage of such type of process and chewing gum is however, that the different ingredients, such as encapsulated flavor, active ingredients, etc. may be more or less destroyed or degraded by the mixing process.

The second type of chewing gum generally benefits of a relatively gentle handling of vulnerable additives, such as the above mentioned flavors or active ingredients. One disadvantage of such type of chewing gum is however, that the resulting chewing gum tablet may typically disintegrate to easy, especially during the initial chew of the gum.

It has been moreover been recognized within the art of chewing gum manufacturing that the process of compressing a chewing gum on the basis of a pre-processed chewing gum material is complicated for different reasons.

US patent 4,753,805 discloses a method of manufacturing compressed chewing gum on the basis of a pre-processed chewing gum composition. One disadvantage of the disclosed chewing gum manufacturing method is that the chewing gum composition, in order to facilitate the final compression process, requires different additives, referred to as compression aid. Evidently, such additives represent further costs and moreover, the additives become an inherent part of the final obtained chewing gum, thereby affecting the final texture or taste.

US patent 4,000,321 discloses a further method of obtaining a compressed chewing gum on the basis of a pre-processed gum base granulate. One disadvantage of the disclosed method is that the applied granulates needs to be heated in order to become self-adhered together. In this way, both the active ingredients may become degenerated and moreover, the texture may become too "solid-like".

Documents EP 0 151 344, US 5,017,385 and US 4,737 366 disclose drawing gum compositions in which granules are pressed together.

It is one object of the invention to provide a chewing gum compression chewing gum composition, which, when processed by means of compression provides a texture like conventionally mixed chewing gum.

### Summary of the invention

The invention relates to a compressed chewing gum tablet comprising a chewing gum center, said gum center comprising a compression of gum base granules and chewing gum additives, said chewing gum additives comprising sweeteners and flavors, at least a first part of said gum base granules comprising flavor or active ingredients incorporated in the gum base and at least a second part of said gum base granules comprises granules of conventional gum base.

According to the invention, conventional gum base refers to a gum base comprising water insolvent parts, and more specifically conventional gum base refers to a gum base which has not been mixed with flavors or active ingredients.

According to the invention, an advantageous combination of standard chewing gum and compression chewing gum has been obtained. According to the invention, an advantageous combination of pre-release and post-release may be obtained. Moreover, a combination of a good early mouth-feel and post mouth feel may be obtained.

Chewing gum additives may, according to the invention broadly refer to sweeteners, flavors, acids, colors, active ingredients, cooling agents, freeze-dried fruit, etc. Moreover, the applied ingredients may be encapsulated.

In accordance with the invention, the chewing gum base components which are used herein may include one or more resinous compounds contributing to obtain the desired masticatory properties and acting as plasticizers for the elastomers.

The balance between the pre-mix and granulated gum base in the mix may vary significantly from application to application, depending upon the desired consistence of the final compressed chewing gum.

One of several advantages according to the invention when applying a multi-string process, is that one of the strings, e.g. the one referred to as pre-mix may comprise a product specifier and the second string may comprise a universal base mix, typically a granulate, that may be applied for every two string process. In this way, different pre-mixes of e.g. flavor or active ingredients may constitute the end product defining mix string.

This feature represents a further advantage in the sense, that the universal gum base mix, due to the fact that it is basically free of flavors or other base modifying ingredients, is relatively stable and may be manufactured and stored relatively robust to environmental influences such as humidity and temperature when compared to the resulting pre-mix granulate comprising the incorporated flavor.

According to a further embodiment of the invention, it is moreover possible to adjust and control the flavor release of the resulting chewing gum as a balance between early release - primarily obtained by flavor and sweeteners added when compressing the combined granulate into the final chewing gum- and late release - primarily obtained by flavor, which has been incorporated into the elastomers of the gum base during the pre-mix stage.

The balance between the pre-mix and the granulated gum base in the mix may vary significantly from application to application depending on the desired flavor release of the final chewing gum and the concentration of the flavor in the pre-mix.

According to a further embodiment of the invention the chew profile may advantageously be adjusted when combining the pre-mix gum base(s) with the second string gum base(s).

When at least a first part of said gum base granules comprising flavor resistant resin, a further advantageous embodiment of the invention has been obtained.

When said at least a first part of said gum base granules comprising synthetic resin, a further advantageous embodiment of the invention has been obtained.

When said synthetic resin comprises polyvinyl acetate, vinyl acetate-vinyl laurate copolymers and mixtures thereof, a further advantageous embodiment of the invention has been obtained.

When said at least a first part of said gum base granules being substantially wax-free, a further advantageous embodiment of the invention has been obtained.

When said chewing gum tablet having a water content of less than 5% by weight, preferably of less than 3% by weight, a further advantageous embodiment of the invention has been obtained.

Typically a relatively low amount of water content is preferred, e.g. due to the fact that the obtained compressed chewing gum may become easier to handle. Moreover, when applying active ingredients, especially non encapsulated active ingredients, undesired chemical reactions may be invoked if the water content is too high.

According to the invention, it has moreover been established that an acceptable texture may be obtained even when operating with extremely low water content, even less than 1.0% by weight.

When said gum center being substantially free of compression aid compounds, a further advantageous embodiment of the invention has been obtained.

When said at least a second part of said gum base granules being tackiness moderated, a further advantageous embodiment of the invention has been obtained. The tackiness moderation may e.g. be obtained by the application of natural resins or for example wax.

When said at least a second part of said gum base granules comprising natural resins, a further advantageous embodiment of the invention has been obtained.

When said at least a second part of said gum base granules comprising wax, a further advantageous embodiment of the invention has been obtained.

When wherein said moderated tackiness being obtained by means of at least one natural resin incorporated in at least a part of the gum base granules, a further advantageous embodiment of the invention has been obtained.

When the compressed chewing gum tablet comprises about 3 % to 50 % by weight of natural resins, preferably about 5% to 40% by weight, a further advantageous embodiment of the invention has been obtained.

When the compressed chewing gum tablet comprises about 0.5% to 30% by weight of elastomers, preferably about 5% to 25% by weight, a further advantageous embodiment of the invention has been obtained.

When the compressed chewing gum tablet comprises about 0.1 % to 15 % by weight of flavoring agents, preferably about 0.8 % to 5 % by weight, a further advantageous embodiment of the invention has been obtained.

When the natural resins provides an improved and sticky texture of the tablet, a further advantageous embodiment of the invention has been obtained.

When the barrier layer comprises e.g. lubricants, anti-adherents and glidants, a further advantageous embodiment of the invention has been obtained.

When the barrier layer comprises magnesium stearate, a further advantageous embodiment of the invention has been obtained.

When the barrier layer comprises metallic stearates, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil and mixtures thereof, a further advantageous embodiment of the invention has been obtained.

When the gum center is substantially free of lubricants, anti-adherents and glidants, a further advantageous embodiment of the invention has been obtained.

Moreover, the invention relates to a chewing gum granulate where at least a part of the chewing gum granulate particles being incorporated with flavor and comprising gum base made on the basis of synthetic resins and where at least a part of the chewing gum granulate particles comprising gum base made on the basis of natural resins.

Moreover, the invention relates to a method of providing a compressed chewing gum comprising the steps of
- mixing at least one elastomer and at least one plasticizer into a first homogenous gum base,
- incorporating an amount flavor into said first gum base, preferably by mechanically mixing,
- granulating said flavor incorporated gum base,
- mixing at least one elastomer and at least one plasticizer into a second homogenous gum base,
- granulating said second gum base,
- blending said first and said second gum base during addition of further chewing gum additives and
- compressing the mixture into a tablet.

According to an embodiment of the invention, the moderated tackiness of the gum base granules should simply be enough to keep the compressed gum base granules together, especially during the initial chew.

Moreover, according to the invention, it has been recognized that controlling of tackiness, preferably established by means of natural resins facilitates a more freely selected group of tablet shapes.

According to the invention it is now possible to obtain a chewing gum tablet, made by means of compression of a gum base granulate and chewing gum additives, having an acceptable and improved immediate initial texture.

Evidently, according to the invention, further additives may be added to the gum base, e.g. during mixing or after mixing.

Moreover, according to the invention, it has been recognized that the natural resin facilitates an advantageous overall flavor release when the compressed chewing gum tablet is chewed. This may partly be due to the fact that the initial chewing of the gum tablet results in an immediate release of flavor particle and at the same time, that a part of the dissolved flavor particles reacts or become incorporated into the chewing gum base.

The last part of the flavor release results in prolonging of the overall flavor release time.

Moreover, a further advantage of the chewing gum tablet according to the invention is that the tablet may be temporarily stored prior to the final processing such as coating and the final packaging.

The upper limit of the desired tackiness is reached, when the gum base granules can no longer be processed by conventional compression techniques.

Moreover, according to the invention, it has been recognized that the natural resin facilitates an advantageous overall flavor release when the compressed chewing gum tablet is chewed. This may partly be due to the fact that the initial chewing of the gum tablet results in an immediate release of flavor particle and at the same time, that a part of the dissolved flavor particles reacts or become incorporated into the chewing gum base.

Moreover, according to an embodiment of the invention, the applied layer may form or form part of a humidity barrier. Due to the fact that relatively low water content is preferred according to an embodiment of the invention, the tablet should preferably be protected against too much absorption of humidity from the air.

### Figures

The invention will be described in the following with reference to the figures in which
- fig. 1: illustrates a chewing gum tablet according to the invention and
- fig. 2: illustrates a flowchart of a chewing gum manufacturing method according to one embodiment of the invention

### Detailed description

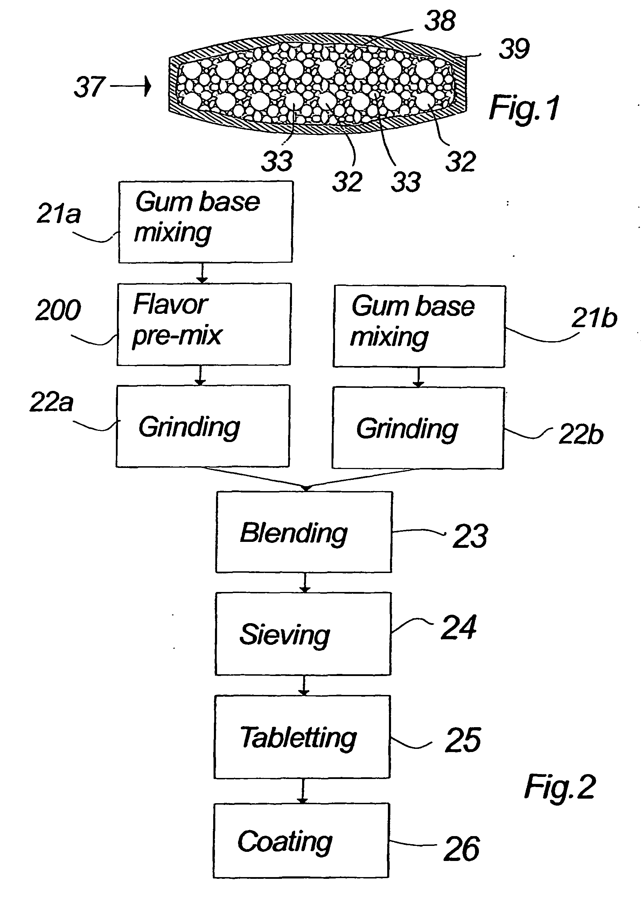

Fig. 1 illustrates a chewing gum tablet according to the invention.

The fig. 1 illustrates a chewing gum tablet made on the basis of compressed gum base granulates.

The gum base granulates are made on the basis of a gum base. As used herein, the expressions "gum base" refers in general to the water insoluble part of the chewing gum which typically constitutes 10 to 90% by weight including the range of 15 -50% by weight of the total chewing gum formulation. Chewing gum base formulations typically comprises one or more elastomeric compounds which may be of synthetic or natural origin, one or more resinous compounds which may be of synthetic or natural origin, fillers, softening compounds and minor amounts of miscellaneous ingredients such as antioxidants and colorants, etc.

Although compressed, the illustrated final chewing gum 37 basically comprises gum base granules 33 and chewing gum additives 32. The chewing gum 37 comprises a gum center 38 encapsulated by a barrier layer 39. Chewing gum additives may within the scope of the invention refer to several chewing gum additives, such as sweeteners, flavor, acids, active ingredients, etc.

The composition of chewing gum base formulations, which are admixed with chewing gum additives as defined below, can vary substantially depending on the particular product to be prepared and on the desired masticatory and other sensory characteristics of the final product. However, typical ranges (weight%) of the above gum base components are: 5 to 50% by weigth elastomeric compounds, 5 to 55% by weight elastomer plasticizers, 0 to 50% by weight filler/texturiser, 5 to 35% by weight softener and 0 to 1% by weight of miscellaneous ingredients such as antioxidants, colourants, etc.

Although not illustrated, a barrier layer may preferably be applied during or prior to the processing of the tablet. The barrier layer, e.g. Mg Stearate, forms an outer barrier of the gum tablet.

Magnesium stearate may e.g. be applied as a pulverized parting compound.

The barrier layer may be added to the final tablet, for example by depositing dosed quantities of pulverized lubricants and parting compounds on the materials contacting surfaces of pressing tools of tabletting machines.

The barrier layer comprises metallic stearates, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil and mixtures thereof.

One of the functions of a barrier layer, if applied, is to prevent sticking to the pressing tools of the tablet compression machine and moreover, consequently, facilitate an increasing of the tackiness of the gum center.

Further layers may be applied to the tablet, such as traditional coatings.

Fig. 2 shows a typical flowchart, illustrating the major steps of one of several applicable manufacturing process within the scope of the invention.

In steps 21 a and 21 b, at least two different suitable gum bases are prepared according to the prescriptions of the invention.

In step 200, at least one of the gum bases is pre-mixed or teared with flavor and sweetener. This gum base may also be referred to as a pre-mix in the following.

One of the gum bases, - here the gum base mixed in step 21b, comprises a conventional gum base adapted for chewing gum granulates.

The pre-mixing of flavors or active ingredients in step 200 may e.g. be performed by means of conventional mixers, e.g. a Z-blade mixer, during no or preferably relatively little added heating and substantially under atmospheric pressure. Preferably, the purely mechanically pre-mixing (also referred to as tearing) should be performed sufficiently enough to result in a homogeneous blend of the flavor and/or active ingredients into the gum base.

Typical duration in time of mixing may be between few minutes op to e.g. 30 minutes. Evidently, according to the invention, other temperatures, pressures, duration in time and mixing methods may be applied for the purpose of mixing active ingredients and/or flavors into the gum base and thereby the gum base granulate applied for the subsequent compression.

In step 22a, the pre-mixed gum base is grinded. The grinding may be performed by means of well-known techniques. One of those techniques implies an initial cooling of the gum base immediately prior to granulation. If the consistence of the gum base allows so, the provided gum base may be grinded at room temperature.

Likewise, in step 22b, the other gum base is grinded separately, e.g. by the same methods as above-described.

According to an advantageous embodiment of the invention, bulk-sweeteners may advantageously be applied as a grinding aid. Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

In step 23, the gum base granulate is blended with suitable chewing gum additives.

The resulting blend comprises a blend of gum base granulates pre-mixed with flavor and optionally further ingredients, such as sweeteners, originating from the pre-mix steps 21a, 200, and 22a, and a gum base granulate originating from the process steps 21b and 22b.

Moreover, the blend obtained in step 23 comprises further chewing gum additives.

In the present context, chewing gum additives include bulk sweeteners, high intensity sweeteners, flavouring agents, softeners, emulsifiers, colouring agents, binding agents, acidulants, fillers, antioxidants and other components such as pharmaceutically or biologically active substances, that confer desired properties to the finished chewing gum product.

Examples of suitable sweeteners are listed below.

Suitable bulk sweeteners include e.g. both sugar and non-sugar components. Bulk sweeteners typically constitute from about 5 to about 95% by weight of the chewing gum, more typically about 20 to about 80% by weight such as 30 to 60% by weight of the gum.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, com syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High intensity artificial sweetening agents can also be used alone or in combination with the above sweeteners. Preferred high intensity sweeteners include, but are not limited to sucralose, aspartame, salts of acesulfame, alitame, saccharin and its salts, neotame, cyclamic acid and its salts, glycyrrhizin, dihydrochalcones, thaumatin, monellin, sterioside and the like, alone or in combination. In order to provide longer lasting sweetness and flavour perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fibre extrusion may be used to achieve desired release characteristics. Encapsulation of sweetening agents can also be provided e.g. using as the encapsulation agent another chewing gum component such as a resinous compound.

Usage level of the artificial sweetener will vary considerably depending e.g. on factors such as potency of the sweetener, rate of release, desired sweetness of the product, level and type of flavour used and cost considerations. Thus, the active level of artificial sweetener may vary from about 0.02 to about 8% by weight When carriers used for encapsulation are included, the usage level of the encapsulated sweetener will be proportionately higher. Combinations of sugar and/or non-sugar sweeteners can be used in the chewing gum formulation processed in accordance with the invention. Additionally, the softener may also provide additional sweetness such as with aqueous sugar or alditol solutions.

If a low calorie gum is desired, a low caloric bulking agent can be used. Examples of low caloric bulking agents include polydextrose, Raftilose, Raftilin, Inuline, fructooligosaccharides (NutraFlora^{®}), palatinose oligosaccharided; guar gum hydrolysates (e.g. Sun Fiber^{®}) or indigestible dextrins (e.g. Fibersol^{®}). However, other low calorie-bulking agents can be used.

Further chewing gum additives which may be included in the chewing gum mixture processed in the present process include surfactants and/or solubilisers, especially when pharmaceutically, cosmetically or biologically active ingredients are present. As examples of types of surfactants to be used as solubilisers in a chewing gum composition according to the invention reference is made to H.P. Fiedler, Lexikon der Hilfstoffe für Pharmacie, Kosmetik und Angrenzende Gebiete, page 63-64 (1981) and the lists of approved food emulsifiers of the individual countries. Anionic, cationic, amphoteric or non-ionic solubilisers can be used. Suitable solubilisers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. The expression "solubiliser" is used in the present text to describe both possibilities, the solubiliser used must be suitable for use in food and/or medicine.

In the presence of an active ingredient the chewing gum may preferably also comprise a carrier known in the art.

One significant advantage of the present process is that the temperature throughout the entire operation can be kept at a relatively low level such as it will be described in the following. This is an advantageous feature with regard to preserving the aroma of added flavouring components which may be prone to deterioration at higher temperatures. Aroma agents and flavouring agents which are useful in a chewing gum produced by the present process are e.g. natural and synthetic flavourings (including natural flavourings) in the form of freeze-dried natural vegetable components, essential oils, essences, extracts, powders, including acids and other substances capable of affecting the taste profile. Examples of liquid and powdered flavourings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

In one preferred embodiment, the flavour is one or more natural flavouring agent(s) which is/are freeze-dried, preferably in the form of a powder, slices or pieces of combinations thereof. The particle size of such agent may be less than 3 mm, such as less than 2 mm, more preferred less than 1 mm, calculated as the longest dimension of the particle. The natural flavouring agent may also be in a form where the particle size is from about 3 µm to 2 mm, such as from 4 µm to 1 mm. Preferred natural flavouring agents include seeds from a fruit e.g. from strawberry, blackberry and raspberry.

Various synthetic flavours, such as mixed fruit flavour may also be used according to the present invention. As indicated above, the aroma agent may be used in quantities smaller than those conventionally used. The aroma agents and/or flavours may be used in an amount from 0.01 to about 30% by weight of the final product depending on the desired intensity of the aroma and/or flavour used. Preferably, the content of aroma/flavour is in the range of from 0.2 to 3% by weight of the total composition.

According to the invention, encapsulated flavors or active ingredients, may be added to the final blend, e.g. in step 23 of fig. 2, prior to compression.

Different methods of encapsulating flavors or active ingredients, which may both refer to flavors or active ingredients mixed into the gum base and flavors or active ingredients compressed into the chewing gum may e.g. include Spray drying, Spray cooling, Film coating, Coascervation, Double emulsion method (Extrusion technology) or Prilling

Materials to be used for the above mentioned encapsulation methods may e.g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Active ingredients may be added to chewing gum. Preferably, these ingredients should be added subsequent to any significant heating or mixing: In other words, the active ingredients, should preferably be added immediately prior to the compression of the final tablet.

Referring to the process illustrated in fig. 2, the adding of active ingredients may be cautiously blended with pre-mixed gum base granulates and further desired additives, immediately prior to the final compression of the tablet.

Examples of suitable active ingredients are listed below.

In one embodiment the chewing gum according to the invention comprises a pharmaceutically, cosmetically or biologically active substance. Examples of such active substances, a comprehensive list of which is found e.g. in WO 00/25598, which is incorporated herein by reference, include drugs, dietary supplements, antiseptic agents, pH adjusting agents, anti-smoking agents and substances for the care or treatment of the oral cavity and the teeth such as hydrogen peroxide and compounds capable of releasing urea during chewing. Examples of useful active substances in the form of antiseptics include salts and derivatives of guanidine and biguanidine (for instance chlorhexidine diacetate) and the following types of substances with limited water-solubility: quaternary ammonium compounds (e.g. ceramine, chloroxylenol, crystal violet, chloramine), aldehydes (e.g. paraformatdehyde), derivatives of dequaline, polynoxyline, phenols (e.g. thymol, p-chlorophenol, cresol), hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. also Martindale, The Extra Pharmacopoeia, 28th edition, page 547-578; metal salts, complexes and compounds with limited water-solubility, such as aluminium salts, (for instance aluminium potassium sulphate AlK(SO₄)₂,12H₂O) and salts, complexes and compounds of boron, barium, strontium, iron, calcium, zinc, (zinc acetate, zinc chloride, zinc gluconate), copper (copper chloride, copper sulphate), lead, silver, magnesium, sodium, potassium, lithium, molybdenum, vanadium should be included; other compositions for the care of mouth and teeth: for instance; salts, complexes and compounds containing fluorine (such as sodium fluoride, sodium monofluorophosphate, aminofluorides, stannous fluoride), phosphates, carbonates and selenium. Further active substances can be found in J. Dent.Res. Vol. 28 No. 2, page 160-171,1949.

Examples of active substances in the form of agents adjusting the pH in the oral cavity include: acids, such as adipinic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulphates or oxides of sodium, potassium, ammonium, magnesium or calcium, especially magnesium and calcium.

Active ingredients may comprise the below mentioned compounds or derivates thereof but are not limited thereto: Acetaminophen, Acetylsalicylsyre Buprenorphine Bromhexin Celcoxib Codeine, Diphenhydramin, Diclofenac, Etoricoxib, Ibuprofen, Indometacin, Ketoprofen, Lumiracoxib, Morphine, Naproxen, Oxycodon, Parecoxib, Piroxicam, Pseudoefedrin, Rofecoxib, Tenoxicam, Tramadol, Valdecoxib, Calciumcarbonat, Magaldrate, Disulfiram, Bupropion, Nicotine, Azithromycin, Clarithromycin, Clotrimazole, Erythromycin, Tetracycline, Granisetron, Ondansetron, Prometazin, Tropisetron, Brompheniramine, Ceterizin, leco-Ceterizin, Chlorcyclizine, Chlorpheniramin, Chlorpheniramin, Difenhydramine, Doxylamine, Fenofenadin, Guaifenesin, Loratidin, des-Loratidin, Phenyltoloxamine, Promethazin, Pyridamine, Terfenadin, Troxerutin, Methyldopa, Methylphenidate, Benzalcon. Chloride, Benzeth. Chloride, Cetylpyrid. Chloride, Chlorhexidine, Ecabet-sodium, Haloperidol, Allopurinol, Colchinine, Theophylline, Propanolol, Prednisolone, Prednisone, Fluoride, Urea, Actot, Glibenclamide, Glipizide, Metformin, Miglitol, Repaglinide, Rosiglitazone, Apomorfin, Cialis, Sildenafil, Vardenafil, Diphenoxylate, Simethicone, Cimetidine, Famotidine, Ranitidine, Ratinidine, cetrizin, Loratadine, Aspirin, Benzocaine, Dextrometorphan, Phenylpropanolamine, Pseudoephedrine, Cisapride, Domperidone, Metoclopramide, Acyclovir, Dioctylsulfosucc., Phenolphtalein, Almotriptan, Eletriptan, Ergotamine, Migea, Naratriptan, Rizatriptan, Sumatriptan, Zolmitriptan, Aluminium salts, Calcium salts, Ferro salts, Ag-salts, Zinc-salts, Amphotericin B, Chlorhexidine, Miconazole, Triamcinolonacetonid, Melatonine, Phenobarbitol, Caffeine, Benzodiazepiner, Hydroxyzine, Meprobamate, Phenothiazine, Buclizine, Brometazine, Cinnarizine, Cyclizine, Difenhydramine, Dimenhydrinate, Buflomedil, Amphetamine, Caffeine, Ephedrine, Orlistat, Phenylephedrine, Phenylpropanolamin, Pseudoephedrine, Sibutramin, Ketoconazole, Nitroglycerin, Nystatin, Progesterone, Testosterone, Vitamin B12, Vitamin C, Vitamin A, Vitamin D, Vitamin E, Pilocarpin, Aluminiumaminoacetat, Cimetidine, Esomeprazole, Famotidine, Lansoprazole, Magnesiumoxide, Nizatide and or Ratinidine.

The invention is suitable for increased or accelerated release of active agents selected among the group dietary supplements, oral and dental compositions, antiseptic agents, pH adjusting agents, anti-smoking agents, sweeteners, flavourings, aroma agents or drugs. Some of those will be described below.

The active agents to be used in connection with the present invention may be any substance desired to be released from the chewing gum. The active agents, for which a controlled and/or accelerated rate of release is desired, are primarily substances with a limited water-solubility, typically below 10 g/100 ml inclusive of substances which are totally water-insoluble. Examples are medicines, dietary supplements, oral compositions, anti-smoking agents, highly potent sweeteners, pH adjusting agents, flavourings etc.

Other active ingedients are, for instance, paracetamol, benzocaine, cinnarizine, menthol, carvone, coffeine, chlorhexidine-di-acetate, cyclizine hydrochloride, 1,8-cineol, nandrolone, miconazole, mystatine, aspartame, sodium fluoride, nicotine, saccharin, cetylpyridinium chloride, other quaternary ammoniumcompounds, vitamin E, vitamin A, vitamin D, glibenclamide or derivatives thereof, progesterone, acetylsalicylic acid, dimenhydrinate, cyclizine, metronidazole, sodium hydrogencarbonate, the active components from ginkgo, the active components from propolis, the active components from ginseng, methadone, oil of peppermint, salicylamide, hydrocortisone or astemizole.

Examples of active agents in the form of dietary supplements are for instance salts and compounds having the nutritive effect of vitamin B2 (riboflavin), B12, folinic acid, niacine, biotine, poorly soluble glycerophosphates, amino acids, the vitamins A, D, E and K, minerals in the form of salts, complexes and compounds containing calcium, phosphorus, magnesium, iron, zinc, copper, iodine, manganese, chromium, selenium, molybdenum, potassium, sodium or cobalt.

Furthermore, reference is made to lists of nutritients acccepted by the authorities in different countries such as for instance US code of Federal Regulations, Title 21, Section 182.5013.182 5997 and 182.8013-182.8997.

Examples of active agents in the form of compounds for the care or treatment of the oral cavity and the teeth, are for instance bound hydrogen peroxide and compounds capable of releasing urea during chewing.

Examples of active agents in the form of antiseptics are for instance salts and compounds of guanidine and biguanidine (for instance chlorhexidine diacetate) and the following types of substances with limited water-solubility: quaternary ammonium compounds (for instance ceramine, chloroxylenol, crystal violet, chloramine), aldehydes (for instance paraformaldehyde), compounds of dequaline, polynoxyline, phenols (for instance thymol, para chlorophenol, cresol) hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. furthermore Martindale, The Extra Pharmacopoeia, 28th edition, page 547-578; metal salts, complexes and compounds with limited water-solubility, such as aluminium salts, (for instance aluminium potassium sulfate AlK(SO₄)₂,12H₂O) and furthermore salts, complexes and compounds of boron, barium, strontium, iron, calcium, zinc, (zinc acetate, zinc chloride, zinc gluconate), copper (copper chloride, copper sulfate), lead, silver, magnesium, sodium, potassium, lithium, molybdenum, vanadium should be included; other compositions for the care of mouth and teeth: for instance; salts, complexes and compounds containing fluorine (such as sodium fluoride, sodiummono-fluorophosphate, aminofluorides, stannous fluoride), phosphates, carbonates and selenium.

Cf. furthermore J. Dent.Res. Vol. 28 No. 2, page 160-171, 1949, wherein a wide range of tested compounds is mentioned.

Examples of active agents in the form of agents adjusting the pH in the oral cavity include for instance: acceptable acids, such as adipinic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulfates or oxides of sodium, potassium, ammonium, magnesium or calcium, especially magnesium and calcium.

Examples of active agents in the form of anti-smoking agents include for instance: nicotine, tobacco powder or silver salts, for instance silver acetate, silver carbonate and silver nitrate.

In a further embodiment, the sucrose fatty acid esters may also be utilised for increased release of sweeteners including for instance the so-called highly potent sweeteners, such as for instance saccharin, cyclamate, aspartame, thaumatin, dihydrocalcones, stevioside, glycyrrhizin or salts or compounds thereof. For increased released of sweetener, the sucrose fatty acids preferable have a content of palmitate of at least 40% such as at least 50%.

Further examples of active agents are medicines of any type.

Examples of active agents in the form of medicines include coffeine, salicylic acid, salicyl amide and related substances (acetylsalicylic acid, choline salicylate, magnesium salicylate, sodium salicylate), paracetamol, salts of pentazocine (pentazocine hydrochloride and pentazocinelactate), buprenorphine hydrochloride, codeine hydrochloride and codeine phosphate, morphine and morphine salts (hydrochloride, sulfate, tartrate), methadone hydrochloride, ketobemidone and salts of ketobemidone (hydrochloride), beta-blockers, (propranolol), calcium antagonists, verapamil hydrochloride, nifedinpine as well as suitable substances and salts thereof mentioned in Pharm. Int., Nov.85, pages 267-271, Barney H. Hunter and Robert L. Talbert, nitroglycerine, erythrityl tetranitrate, strychnine and salts thereof, lidocaine, tetracaine hydrochloride, etorphine hydrochloride, atropine, insulin, enzymes (for instance papain, trypsin, amyloglucosidase, glucoseoxidase, streptokinase, streptodomase, dextranase, alpha amylase), polypeptides (oxytocin, gonadorelin, (LH.RH), desmopressin acetate (DDAVP), isoxsuprine hydrochloride, ergotamine compounds, chloroquine (phosphate, sulfate), isosorbide, demoxytocin, heparin.

Other active ingredients include beta-lupeol, Letigen^{®}, Sildenafil citrate and derivatives thereof.

Dental products include Carbamide, CPP Caseine Phospho Peptide; Chlorhexidine, Chlorhexidine di acetate, Chlorhexidine Chloride, Chlorhexidine di gluconate, Hexetedine, Strontium chloride, Potassium Chloride, Sodium bicarbonate, Sodium carbonate, Fluor containing ingredients, Fluorides, Sodium fluoride, Aluminium fluoride.

Ammonium fluoride, Calcium fluoride, Stannous fluoride, Other fluor containing ingredients Ammonium fluorosilicate, Potasium fluorosilicate, Sodium fluorosilicate, Ammonium monofluorphosphate, Calcium monofluorphosphate, Potassium monofluorphosphate, Sodium monofluorphosphate, Octadecentyl Ammonium fluoride, Stearyl Trihydroxyethyl Propylenediamine Dihydrofluoride,

Vitamins include A, B1, B2, B6, B12, Folin acid, niacin, Pantothensyre, biotine, C, D, E, K. Minerals include Calcium, phosphor, magnesium, iron, Zink, Cupper, lod, Mangan, Crom, Selene, Molybden. Other active ingredients include: Q10^{®}, enzymes. Natural drugs including Ginkgo Biloba, ginger, and fish oil.

The invention also relates to use of migraine drugs such as Serotonin antagonists: Sumatriptan, Zolmitriptan, Naratriptan, Rizatriptan, Eletriptan; nausea drugs such as Cyclizin, Cinnarizin, Dimenhydramin, Difenhydrinat; hay fever drugs such as Cetrizin, Loratidin, pain relief drugs such as Buprenorfin, Tramadol, oral disease drugs such as Miconazol, Amphotericin B, Triamcinolonaceton; and the drugs Cisaprid, Domperidon, Metoclopramid. In a preferred embodiment the invention relates to the release of Nicotine and its salts.

As above mentioned active ingredients and/or flavors may be pre-mixed into the gum base.

When the gum base granules comprises pre-mixed active ingredients, a controlled release of active ingredients may be obtained by means of at least a double active ingredients buffer. The first buffer comprising active ingredients blended into the final mix immediately prior to compression and the second buffer comprising active ingredients blended into the gum base prior to the blending of gum base and gum base additives.

Generally, release of flavor and/or active ingredients may be adjusted by adjustment of the balance between pre-mixed ingredients and the chewing gum additives added prior to compression when carefully blending the gum base granulate with the remaining desired chewing gum additives.

In step 24, the resulting blend is prepared for tabletting by means of sieving.

The degree of sieving depends primarily of how the gum base granulate(s) "reacts" when chewing gum additives are blended together.

If a barrier layer is desired, this may be done in principally two ways.

If suitable, an initial pre-forming of the granulates may be supplemented by spraying the barrier layer at the surface or at least a part of the surface of the pre-formed granulates. This technique and variants thereof may be referred to as an explicit barrier layer depositing.

However, preferably, the barrier layer is established in a more implicit way. This technique and variants thereof may be referred to as implicit barrier layer depositing. This technique implies that the barrier layer compound is sprayed or deposited initially on the contacting surfaces of the pressing tools of a compression machine.

An applicable technique suitable for implicit-barrier layer depositing is disclosed in US patent 5,643,630.

An optional barrier layer may comprise of e.g. lubricants, anti-adherents and glidants.

Magnesium stearate may e.g. be applied as a pulverized parting compound.

The barrier layer may be added to the final tablet for example by depositing dosed quantities of pulverized lubricants and parting compounds on the materials contacting surfaces of pressing tools of tabletting machines.

The barrier layer may be established by means of for example metallic stearates, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil and mixtures thereof.

If no barrier is applied, compression aid, known within the art should be incorporated in the final blend in order to avoid sticking to the compression mechanics.

In step 25, the grinded blend is applied to the pressing tools of a tabletting machine and compressed into chewing gum tablets.

In step 26, which is optional, - but preferred, the tabletted chewing gum is provided with a suitable coating. It should here be noted that the preferred chewing gum tablet according to an embodiment of the invention has a very low water content. Therefore, in order to keep the gum center stable with respect to influence from the surroundings, primarily in the form of humidity, a coating should be applied.

In accordance with the invention, the chewing gum element comprises about I to about 75% by weight of an outer coating applied onto the chewing gum centre. In the present context, a suitable outer coating is any coating that results in an extended storage stability of the compressed chewing gum products as defined above, relative to a chewing gum of the same composition that is not coated. Thus, suitable coating types include hard coatings, film coatings and soft coatings of any composition including those currently used in coating of chewing gum, pharmaceutical products and confectioneries.

According to a preferred embodiment of the invention, film coating is applied to the compressed chewing gum tablet.

One presently preferred outer coating type is a hard coating, which term is used in the conventional meaning of that term including sugar coatings and sugar-free (or sugarless) coatings and combinations thereof. The objects of hard coating is to obtain a sweet, crunchy layer which is appreciated by the consumer and to protect the gum centres for various reasons as. In a typical process of providing the chewing gum centres with a protective sugar coating the gum centres are successively treated in suitable coating equipment with aqueous solutions of crystallisable sugar such as sucrose or dextrose, which, depending on the stage of coating reached, may contain other functional ingredients, e.g. fillers, colours, etc. In the present context, the sugar coating may contain further functional or active compounds including flavour compounds, pharmaceutically active compounds and/or polymer degrading substances.

In the production of chewing gum it may, however, be preferred to replace the cariogenic sugar compounds in the coating by other, preferably crystallisable, sweetening compounds that do not have a cariogenic effect. In the art such coating are generally referred to as sugarless or sugar-free coatings. Presently preferred non-cariogenic hard coating substances include polyols, e.g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively.

In a typical hard coating process as it will be described in details in the following, a syrup containing crystallisable sugar and/or polyol is applied onto the gum centres and the water it contains is evaporated off by blowing with warm, dry air. This cycle must be repeated several times, typically 10 to 80 times, in order to reach the swelling required. The term "swelling" refers to the increase in weight of the products, as considered at the end of the coating operation by comparison with the beginning, and in relation to the final weight of the coated products. In accordance with the present invention, the coating layer constitutes about 1 to about 75% by weight of the finished chewing gum element, such as about 10 to about 60% by weight, including about 15 to about 50% by weight.

In further useful embodiments the outer coating of the chewing gum element of the invention is an element that is subjected to a film coating process and which therefore comprises one or more film-forming polymeric agents and optionally one or more auxiliary compounds, e.g. plasticizers, pigments and opacifiers. A film coating is a thin polymer-based coating applied to a chewing gum centre of any of the above forms. The thickness of such a coating is usually between 20 and 100 µm. Generally, the film coating is obtained by passing the chewing gum centres through a spray zone with atomised droplets of the coating materials in a suitable aqueous or organic solvent vehicle, after which the material adhering to the gum centres is dried before the next portion of coating is received. This cycle is repeated until the coating is complete.

In the present context, suitable film-coating polymers include edible cellulose derivatives such as cellulose ethers including methylcellulose (MC), hydroxyethyl cellulose (HEC), hydroxypropyl cellulose (HPC) and hydroxypropyl methylcellulose (HPMC). Other useful film-coating agents are acrylic polymers and copolymers, e.g. methylacrylate aminoester copolymer or mixtures of cellulose derivatives and acrylic polymers. A particular group of film-coating polymers, also referred to as functional polymers are polymers that, in addition to its film-forming characteristics, confer a modified release performance with respect to active components of the chewing gum formulation. Such release modifying polymers include methylacrylate ester copolymers, ethylcellulose (EC) and enteric polymers designed to resist the acidic stomach environment, yet dissolve readily in the duodenum. The latter group of polymers include: cellulose acetate phtalate (CAP), polyvinyl acetate phtalate (PVAP), shellac, metacrylic acid copolymers, cellulose acetate trimellitate (CAT) and HPMC. It will be appreciated that the outer film coating according to the present invention may comprise any combination of the above film-coating polymers.

In other embodiments, the film coating layer of the chewing gum elements according to the invention comprises a plasticizing agent having the capacity to alter the physical properties of a polymer to render it more useful in performing its function as a film-forming material. In general, the effect of plasticizers will be to make the polymer softer and more pliable as the plasticizer molecules interpose themselves between the individual polymer strands thus breaking down polymer-polymer interactions. Most plasticizers used in film coating are either amorphous or have very little crystallinity. In the present context, suitable plasticizers include polyols such as glycerol, propylene glycol, polyethylene glycol, e.g. the 200-6000 grades hereof, organic esters such as phtalate esters, dibutyl sebacate, citrate esters and thiacetin, oils/glycerides including castor oil, acetylated monoglycerides and fractionated coconut oil.

The choice of film-forming polymer(s) and plasticizing agent(s) for the outer coating of the present chewing gum element is made with due consideration for achieving the best possible barrier properties of the coating in respect of dissolution and diffusion across the film of moisture and gasses.

The film coating of the chewing gum elements may also contain one or more colourants or opacifiers. In addition to providing a desired colour hue, such agents may contribute to protecting the compressed gum base against pre-chewing reactions, in particular by forming a barrier against moisture and gasses. Suitable colourants/pacifiers include organic dyes and their lakes, inorganic colouring agents, e.g. titanium oxide and natural colours such as e.g. β-carotenc.

Additionally, film coatings may contain one or several auxiliary substances such as flavours and waxes or saccharide compounds such as polydextrose, dextrins including maltodextrin, lactose, modified starch, a protein such as gelatine or zein, a vegetable gum and any combination thereof.

In one specific embodiment the chewing gum centre is in the form of a stick which is provided on one or both sides with an edible film comprising alternate layers of a coating of a water soluble film forming agent, e.g. a cellulose derivative, a modified starch, a dextrin, gelatine, zein, a vegetable gum, a synthetic polymer and any combination thereof, and a wax such as beeswax, carnauba wax, microcrystalline wax, paraffin wax and combinations thereof.

It is also an aspect of the present invention that the outer coating of the chewing gum element can contain one or more pharmaceutically or cosmetically components including those mentioned hereinbefore.

Accordingly, in further embodiments, the above hard-coated or film-coated chewing gum element of the invention is an element where the outer coating comprises at least one additive component selected from a binding agent, a moisture absorbing component, a film forming agent, a dispersing agent, an antisticking component, a bulking agent, a flavouring agent, a colouring agent, a pharmaceutically or cosmetically active component, a lipid component, a wax component, a sugar and an acid. If it is desired to defer the effect of any of these additive components in the outer coating until mastication of the chewing gum, such components may, in accordance with the invention be encapsulated using any conventional encapsulation agent such as e.g. a protein including gelatine and soy protein, a cellulose derivative including any of those mentioned above, a starch derivative, edible synthetic polymers and lipid substances, the latter optionally in the form of liposome encapsulation.

In other embodiments, the chewing gum element according to the invention is provided with an outer coating in the form generally described in the art as a soft coating. Such soft coatings are applied using conventional methods and may advantageously consist of a mixture of a sugar or any of the above non-cariogenic, sugar-less sweetening compounds, and a starch hydrolysate.

Again, it should be noted that the above-described coating is optional or that it may be postponed until it fits into the last part of the manufacturing process due to the fact that the applied barrier layer is also acting as a complete or at least a partial barrier to transfer of humidity from the environment into the tablet.

Generally with respect to the gum base formulations applicable within the scope of the invention, useful synthetic elastomers include, but are not limited to, synthetic elastomers listed in Food and Drug Administration, CFR, Title 21, Section 172,615, the Masticatory Substances, Synthetic) such as polyisobutylene. e.g. having a gas pressure chromatography (GPC) average molecular weight in the range of about 10,000 to about 1,000,000 including the range of 50,000 to 80,000, isobutylene-isoprene copolymer (butyl elastomer), styrene-butadiene copolymers e.g. having styrene-butadiene ratios of about 1:3 to about 3:1, polyvinyl acetate (PVA), e.g. having a GPC average molecular weight in the range of 2,000 to about 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to about 50% by weight such as 10 to 45% by weight of the copolymer, and combinations hereof.

It is common in the industry to combine in a gum base a synthetic elastomer having a high molecular weight and a low-molecular-weight elastomer. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Particularly interesting elastomeric or resinous polymer compounds which advantageously can be used in a process according to the invention include polymers which, in contrast to currently used elastomers and resins, can be degraded physically, chemically or enzymatically in the environment after use of the chewing gum, thereby giving rise to less environmental pollution than chewing gums based on non-degradable polymers, as the used degradable chewing gum remnants will eventually disintegrate and/or can be removed more readily by physical or chemical means from the site where it has been dumped.

In accordance with the invention, the chewing gum base components which are used herein may include one or more resinous compounds contributing to obtain the desired masticatory properties and acting as plasticizers for the elastomers of the gum base composition. In the present context, useful elastomer plasticizers include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of elastomer plasticizers will vary depending on the specific application, and on the type of elastomer(s) being used.

A chewing gum base formulation may, if desired, include one or more fillers/texturisers including as examples, magnesium and calcium carbonate, sodium sulphate, ground limestone, silicate compounds such as magnesium and aluminium silicate, kaolin and clay, aluminium oxide, silicium oxide, talc, titanium oxide, mono-, di- and tri-calciwn phosphates, cellulose polymers, such as wood, and combinations thereof.

The fillers/texturisers may also include natural organic fibres such as fruit vegetable fibres, grain, rice, cellulose and combinations thereof.

A gum base formulation may, in accordance with the present invention comprise one or more softeners e.g. sucrose polyesters including those disclosed in WO 00/25598, which is incorporated herein by reference, tallow, hydrogenated fat including tallow, hydrogenated and partially hydrogenated vegetable oils, cocoa butter, glycerol monostearate, glycerol triacetate, lecithin, mono-, di- and triglycerides, acetylated monoglycerides, fatty acids (e.g. stearic, palmitic, oleic and linoleic acids), and combinations thereof. As used herein the term "softener" designates an ingredient, which softens the gum base or chewing gum formulation and encompasses waxes, fats, oils, emulsifiers, surfactants and solubilisers.

To soften the gum base further and to provide it with water binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Mono- and diglycerides of edible fatty acids, lactic acid esters and acetic acid esters of mono and diglycerides of edible fatty acids, acetylated mono and diglycerides, sugar esters of edible fatty acids, Na-, K-, Mg- and Ca-stearates, lecithin, hydroxylated lecithin and the like are examples of conventionally used emulsifiers which can be added to the chewing gum base. In case of the presence of a biologically or pharmaceutically active ingredient as defined below, the formulation may comprise certain specific emulsifiers and/or solubilisers in order to enhance dispersion and release of the active ingredient.

Waxes and fats are conventionally used for the adjustment of the consistency and for softening of the chewing gum base when preparing chewing gum bases. In connection with the present invention, any conventionally used and suitable type of wax and fat may be used, such as for instance rice bran wax, polyethylene wax, petroleum wax (refined paraffin and microcrystalline wax), paraffin, bees' wax, carnauba wax, candelilla wax, cocoa butter, degreased cocoa powder and any suitable oil or fat, as e.g. completely or partially hydrogenated vegetable oils or completely or partially hydrogenated animal fats.

Furthermore, the gum base formulation may, in accordance with the present invention, comprise colorants and whiteners such as FD&C-type dyes and lakes, fruit and vegetable extracts, titanium dioxide and combinations thereof. Further useful chewing gum base components include antioxidants, e.g. butylated hydroxytoluene (BHT), butyl hydroxyanisol (BHA), propylgallate and tocopherols, and preservatives.

### Example 1

The chewing gum as disclosed with reference to fig. 2 was prepared.

It should be emphasized that several other gum base compositions may be applied within the scope of the invention.

The first gum base mechanically mixed in step 21a comprised

| | |
|---|---|
| elastomer: | 17 % by weight |
| synthetic resin: | 28 % by weight |
| fat/fillers: | 55 % by weight |
| wax: | 0 % by weight |

In step 200, the first gum bass was pre-mixed with a menthol flavor.

The first gum base comprises approximately 10.3% by weight of the complete blend obtained in step 23.

During granulation in step 22a of the first gum base granulate, a sweetener, sorbitol was added 50:50. In other words, sorbitol comprises approximately 10.3% by weight of the complete blend obtained in step 23. The granulation was performed during cooling.

The second gum base mechanically mixed in step 21b comprised

| | |
|---|---|
| elastomer: | 19 % by weight |
| natural resin: | 20 % by weight |
| synthetic resin: | 20 % by weight |
| fat/fillers: | 26 % by weight |
| wax: | 15 % by weight |

The second gum base comprises approximately 25.6 % by weight of the complete blend obtained in step 23.

During granulation in step 22b of the second gum base granulate, a sweetener, sorbitol was added 50:50. In other words, sorbitol comprises approximately 25.6 % by weight of the complete blend obtained in step 23. The granulation was performed during cooling.

In step 23, both granulates are blended together with an amount of further sweetener, again sorbitol, approximately 18.6 % by weight of the complete blend obtained in step 23, further flavor additive, approximately 1.4 % by weight of the complete blend obtained in step 23 and finally so-called flavor beads and high intensity sweeteners were added. The high intensity sweeteners comprises about 0.15% of aspartame + 0.15% of acesulfame K = 0.3% in total by weight.

Finally, the grinded blend was compressed into a chewing gum. The resulting chewing gum has two significant features, i.e. a double-flavor-release buffer, and a double texture function.

Moreover, the same double-active ingredient buffer may be established in the same way, i.e. by incorporating a part of the active ingredients in the gum base and adding the rest of the active ingredient immediately prior to compression.

The double release buffer was immediately noted by a test panel testing the obtained chewing gum, determining that the double release was obtained, when chewing the chewing gum, an initial release dominated by the additives added in step 23, and a subsequent release dominated by the flavor incorporated in the first gum base granulate.

Moreover, the texture of the chewing gum was determined to be surprisingly impressing compared to that of conventional compressed chewing gum. Especially, the initial chew - texture was noted to be impressing.

Finally, it should be mentioned that although only two gum bases has been described above almost any number of different gum bases may be applied within the scope of the invention.

It should be noted that the first process string, here step 21a, 200, 22a, defines the mixing and preparation of a flavor incorporated gum base granulate, which is preferably based on a natural resin-free gum base and preferably also wax-free.

Generally, according to the invention, the first gum base should preferably be able to incorporate a relatively large amount of flavor without dissolving. This is obtained relatively easy, e.g. by applying of synthetic resins and avoiding wax in the gum base.

The relatively large amount of flavor pre-mixed into the gum-base in step 200 forms one of two basically different flavor release buffers of a chewing gum. Thus, the pre-mixed flavor are incorporated in a part of the granulates and tends to provide a relatively late release, whereas the flavor additives added in step 23, tends to release quite early, during the initial chew of the chewing gum.

Compared to conventionally mixed chewing gum, the compression of a gum base granulate together with chewing gum additives is a relatively lenient gathering of the final chewing gum, at least with respect to temperature. However, the omission of the thoroughly tearing of the granulate together with the desired additives will, according to conventional chewing gum result in a risk of crumbling and disintegration especially during the initial chew.

According to the invention, the provided chewing gum featuring tacky granules may counteract the initial-chew invoked disintegration to such a degree, that the chewing gum remains non-crumpling until the granules are finally mixed during the chewing of the chewing gum.

According to the invention, encapsulated flavors, also referred to as beads within the art, may be added to the final blend, e.g. in step 23 of fig. 2, prior to compression.

One of several advantages according to the invention when applying a multi-string process, is that one of the strings, e.g. the one referred to as pre-mix may comprise a product specifier and the second string may comprise a universal base mix, typically a granulate, that may be applied for every two string process. In this way, different pre-mixes of e.g. flavor or active ingredients may constitute the end product defining mix string.

This feature represents a further advantage in the sense, that the universal gum base mix, due to the fact that it is basically free of flavors or other base modifying ingredients, is relatively stable and may be manufactured and stored relatively robust to environmental influences such as humidity and temperature when compared to the resulting pre-mix granulate comprising the incorporated flavor.

According to a further embodiment of the invention, it is moreover possible to adjust and control the flavor release of the resulting chewing gum as a balance between early release, primarily obtained by flavor and sweeteners added when compressing the combined granulate into the final chewing gum and late release, primarily obtained by flavor, which has been incorporated into the gum base during the pre-mix stage.

The balance between the pre-mix and the granulated gum base in the mix may vary significantly from application to application depending on the desired flavor release of the final chewing gum and the concentration of the flavor in the pre-mix.

According to a further embodiment of the invention the chew profile may advantageously be adjusted when combining the pre-mix gum base(s) with the second string gum base(s).

The pre-mix string may not only include flavors in conventional means but also include active ingredients, encapsulated or non-encapsulated. When the gum base granules comprises pre-mixed active ingredients, a controlled release of active ingredients may be obtained by means of a at least a double active ingredients buffer, the first buffer comprising active ingredients blended into the final mix immediately prior to compression, the second buffer comprising active ingredients blended into the gum base prior to the blending of gum base and gum base additives.

In this way, the balance between pre-mixed ingredients and normal compressed ingredients, a certain desired balance between early and late release of active ingredients may be obtained.

It should be noted that the balance between the early and late release of active ingredients may advantageously be correlated to the early and late release of flavors, thereby facilitating and optimized masking.

## Claims

1. Compressed chewing gum tablet (37) comprising a chewing gum center (38),
said gum center (38) comprising a compression of gum base granules (32) and chewing gum additives (33),
said chewing gum additives comprising sweeteners and flavors,
at least a first part of said gum base granules (32) comprising flavor or active ingredients incorporated gum base,
at least a second part of said gum base granules (32) comprising granules of conventional gum base.

2. Compressed chewing gum tablet according to claim 1, wherein
said at least a first part of said gum base granules (32) comprising flavor resistant resin.

3. Compressed chewing gum tablet according to claim 1 or 2, wherein
said at least a first part of said gum base granules (32) comprising synthetic resin.

4. Compressed chewing gum tablet according to any of claims 1 to 3, wherein
said synthetic resin comprising polyvinyl acetate, vinyl acetate-vinyl laurate copolymers and mixtures thereof.

5. Compressed chewing gum tablet according to any of claims 1 to 4, wherein
said at least a first part of said gum base granules (32) being substantially wax-free.

6. Compressed chewing gum tablet according to any of claims 1 to 5, wherein
said chewing gum tablet having a water content of less than 5% by weight, preferably of less than 3% by weight, even more preferably less than 1% by weight.

7. Compressed chewing gum tablet according to any of claims 1 to 6, wherein
said gum center (38) being substantially free of compression aid compounds.

8. Compressed chewing gum tablet according to any of claims 1 to 7, wherein
said at least a second part of said gum base granules (32) being tackiness moderated.

9. Compressed chewing gum tablet according to any of claims 1 to 8, wherein
said at least a second part of said gum base granules (32) comprising natural resins.

10. Compressed chewing gum tablet according to any of claims 1 to 9, wherein
said at least a second part of said gum base granules (32) comprising wax.

11. Compressed chewing gum tablet according to any of claims 1 to 10, wherein
said moderated tackiness being obtained by means of at least one natural resin incorporated in at least a part of the gum base granules (32)

12. Compressed chewing gum tablet according to any of claims 1 to 11, wherein
the compressed chewing gum tablet comprises 3 % to 50 % by weight of natural resins, preferably 5% to 40% by weight.

13. Compressed chewing gum tablet according to any of claims 1 to 12, wherein
the compressed chewing gum tablet comprises 0.5% to 30% by weight of elastomers, preferably 5% to 25% by weight.

14. Compressed chewing gum tablet according to any of claims 1 to 13, wherein
the compressed chewing gum tablet comprises 0.1 % to 15 % by weight of flavoring agents, preferably 0.8 % to 5 % by weight.

15. Compressed chewing gum tablet according to any of claims I to 14, wherein
the natural resins provides an improved and sticky texture of the tablet.

16. Compressed chewing gum tablet according to any of claims 1 to 15, wherein
said barrier layer comprises e.g. lubricants, anti-adherents and glidants.

17. Compressed chewing gum tablet according to any of claims 1 to 16, wherein
the barrier layer comprises magnesium stearate.

18. Compressed chewing gum tablet according to any of claims 1 to 17, wherein
said barrier layer comprises metallic stearates, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil and mixtures thereof.

19. Compressed chewing gum tablet according to any of claims 1 to 18, wherein
the gum center is substantially free of lubricants, anti-adherents and glidants.

20. Compressed chewing gum according to any of the claims 1 to 19, wherein said chewing gum additives comprises sweeteners in the amount of from 5 to 95% by weight of the chewing gum, more typically 20 to 80% by weight such as 30 to 60% by weight of the gum.

21. Method of providing a compressed chewing gum comprising the steps of
- mixing at least one elastomer and at least one plasticizer into a first homogenous gum base,
- incorporating an amount flavor into said first gum base, preferably by mechanically mixing,
- granulating said flavor incorporated gum base,
- mixing at least one elastomer and at least one plasticizer into a second homogenous gum base,
- granulating said second gum base,
- blending said first and said second gum base during addition of further chewing gum additives and
- compressing the mixture into a tablet.

22. Method according to claim 21, whereby the granulation is performed during addition of at least one bulk sweetener.

23. Chewing gum granulate wherein
a first part of the chewing gum granulate comprises granules being incorporated with flavor and/or active ingredients
a second part of the chewing gum granulate comprises granules on conventional gum base.

24. Chewing gum granulate according to claim 23, wherein
the first part of the chewing gum granulate comprises synthetic resins.

25. Chewing gum granulate according to claim 23 or 24, wherein
the second part of the chewing gum granules comprises gum base made on the basis of natural resins.

26. Chewing gum granulate according to any of the claims 23 to 25, wherein
the first part of the chewing gum granulate is substantially free of natural resins.

27. Chewing gum granulate according to any of the claims 23 to 26, wherein
the first part of the chewing gum granulate is substantially free of wax.

28. Chewing gum granulate according to any of the claims 23 to 27, wherein
the chewing gum granulate comprises grinding aid, preferably bulk sweeteners.

29. Chewing gum granulate according to the claim 28, wherein
said grinding aid comprises sorbitol.

30. Chewing gum granulate according to any of the claims 23 to 29, wherein
said synthetic resin comprising polyvinyl acetate, vinyl acetate-vinyl laurate copolymers and mixtures thereof.

31. Chewing gum granulate according to any of the claims 23 to 30, wherein the granulate is substantially free of lubricants, anti-adherents and glidants.

## Patentansprüche

1. Komprimierte Kaugummitablette (37), umfassend einen Kaugummikern (38),
wobei der Kaugummikern (38) ein komprimiertes Material aus Gummibasis-Granulat (32) und Kaugummi-Zusätzen (33) umfasst,
wobei die Kaugummi-Zusätze Süßmittel und Geschmacksmittel umfassen,
wobei mindestens ein erster Teil des Gummibasis-Granulats (32) Geschmacksmittel oder aktive Bestandteile in der Gummibasis enthält,
wobei mindestens ein zweiter Teil des Gummibasis-Granulats (32) Granulat von herkömmlicher Gummibasis umfasst.

2. Komprimierte Kaugummitablette nach Anspruch 1, in der der mindestens eine erste Teil des Gummibasis-Granulats (32) ein Geschmacksmittelbeständiges Harz umfasst.

3. Komprimierte Kaugummitablette nach Anspruch 1 oder 2, in der der mindestens eine erste Teil des Gummibasis-Granulats (32) synthetisches Harz umfasst.

4. Komprimierte Kaugummitablette nach Irgendeinem der Ansprüche 1 bis 3, in der das synthetische Harz Polyvinylacetat, Vinylacetat-Vinyllaurat-Copolymere und deren Mischungen umfasst.

5. Komprimierte Kaugummitablette nach irgendeinem der Ansprüche 1 bis 4, in der der mindestens eine erste Teil des Gummibasis-Granulats (32) im Wesentlichen wachsfrei ist.

6. Komprimierte Kaugummitablette nach irgendeinem der Ansprüche 1 bis 5, in der die Kaugummitablette einen Wassergehalt von weniger als 5 Gew.-%, bevorzugt weniger als 3 Gew.-%, noch bevorzugter weniger als 1 Gew.-% aufweist.

7. Komprimierte Kaugummitablette nach irgendeinem der Ansprüche 1 bis 6, in der der Kaugummikern (38) im Wesentlichen frei von Kompressionshilfsstoffverbindungen ist.

8. Komprimierte Kaugummitablette nach Irgendeinem der Ansprüche 1 bis 7, in der der mindestens eine zweite Teil des Gummibasis-Granulats (32) Klebrigkefts-gemäßlgt ist.

9. Komprimierte Kaugummitablette nach irgendeinem der Ansprüche 1 bis 8, in der der mindestens eine zweite Teil des Gummibasis-Granulats (32) natürliche Harze umfasst.

10. Komprimierte Kaugummitablette nach irgendeinem der Ansprüche 1 bis 9, in der der mindestens eine zweite Teil des Kaugummlbasls-Granulats (32) Wachs umfasst.

11. Komprimierte Kaugummitablette nach irgendeinem der Ansprüche 1 bis 10, in der die gemäßigte Klebrigkeit mittels mindestens eines natürlichen Harzes erhalten wird, das in mindestens einem Teil des Gummibasis-Granulats (32) enthalten ist.

12. Komprimierte Kaugummitablette nach irgendeinem der Ansprüche 1 bis 11, in der die komprimierte Kaugummitablette 3 bis 50 Gew.-% natürliche Harze, vorzugsweise 5 bis 40 Gew.-%, umfasst.

13. Komprimierte Kaugummitablette nach irgendeinem der Ansprüche 1 bis 12, in der die komprimierte Kaugummitablette 0,5 bis 30 Gew.-% Elastomere, bevorzugt 5 bis 25 Gew.-%, umfasst.

14. Komprimierte Kaugummitablette nach irgendeinem der Ansprüche 1 bis 13, in der die komprimierte Kaugummitablette 0,1 bis 15 Gew.-% Geschmacksmittel, bevorzugt 0,8 bis 5 Gew.-%, umfasst.

15. Komprimierte Kaugummitablette nach irgendeinem der Ansprüche 1 bis 14, in der die natürlichen Harze eine verbesserte und klebrige Struktur der Tablette bereitstellen.

16. Komprimierte Kaugummitsblette nach irgendeinem der Ansprüche 1 bis 15, In der die Barriereschicht zum Beispiel Schmiermittel, Antihaftmittel und Gleitmittel umfasst.

17. Komprimierte Kaugummitablette nach irgendeinem der Ansprüche 1 bis 16, in der die Barriereschicht Magnesiumstearat umfasst.

18. Komprimierte Kaugummitablette nach irgendeinem der Ansprüche 1 bis 17, in der die Barriereschicht Metallstearate, hydrierte Pflanzenöle, teilhydrierte Pflanzenöle, Polyethylenglycole, Polyoxyethylenmonostearate, Tierfette, Silicate, Silicatdioxid, Talk, Magnesiumstearate, Calciumstearate Kieselpuder, pulverförmige hydrierte Baumwollsamenöle, hydrierte Pflanzenöle, hydriertes Sojaöl und deren Mischungen umfasst.

19. Komprimierte Kaugummitablette nach irgendeinem der Ansprüche 1 bis 18, in der der Gummikern im Wesentlichen frei von Schmiermitteln, Antihaftmitteln und Gleitmitteln ist.

20. Komprimierter Kaugummi nach irgendeinem der Ansprüche 1 bis 19, in der die Kaugummi-Zusätze in einer Menge von 5 bis 95 Gew.-% des Kaugummis, typischer 20 bis 80 Gew.-%, wie beispielsweise 30 bis 60 Gew.-% des Gummis umfassen.

21. Verfahren zur Bereitstellung eines komprimierten Kaugummis, umfassend die Schritte:
- Einmischen mindestens eines Elastomers und mindestens eines Weichmachers in eine erste homogene Gummibasis,
- Einverleiben einer Geschmacksmitteimenge in die erste Gummibasis, bevorzugt durch mechanisches Mischen,
- Granulieren der Geschmacksmittel enthaltenden Gummibasis,
- Einmischen des mindestens einen Elastomers und mindestens einen Weichmachers in eine zweite homogene Gummibasis,
- Granulieren der zweiten Gummibasis,
- Mischen der ersten und zweiten Gummibasis während der Zugabe von weiteren Kaugummi-Zusätzen und
- Komprimieren der Mischung zu einer Tablette.

22. Verfahren nach Anspruch 21, bei dem die Granulierung während der Zugabe mindestens eines massiven Süßmittels durchgeführt wird.

23. Kaugummi-Granulat, in dem
ein erster Teil des Kaugummi-Granulats Granalien umfasst, denen Geschmacksmittel und/oder aktive Bestandteile zugesetzt wurden,
ein zweiter Teil des Kaugummi-Granulats Granalien auf herkömmlicher Gummibasis enthält.

24. Kaugummi-Granulat nach Anspruch 23, in dem der erste Teil des Kaugummi-Granulats synthetische Harze umfasst.

25. Kaugummi-Granulat nach Anspruch 23 oder 24, in dem der zweite Teil des Kaugummi-Granulats Gummibasis umfasst, die auf der Grundlage von natürlichen Harzen hergestellt ist.

26. Kaugummi-Granulat nach irgendeinem der Ansprüche 23 bis 25, in dem der erste Teil des Kaugummi-Granulats im Wesentlichen frei von natürlichen Harzen ist.

27. Kaugummi-Granulat nach irgendeinem der Ansprüche 23 bis 26, in dem der erste Teil des Kaugummi-Granulats im Wesentlichen wachsfrei ist.

28. Kaugummi-Granulat nach irgendeinem der Ansprüche 23 bis 27, in dem das Kaugummigranulat Mahlhilfsmittel, bevorzugt massive Süßmittel umfasst.

29. Kaugummi-Granulat nach Anspruch 28, in dem das Mahlhilfsmittel Sorbit umfasst.

30. Kaugummi-Granulat nach irgendeinem der Ansprüche 23 bis 29, in dem das synthetische Harz Polyvinylacetat, Vinylacetat-Vinyllaurat-Copolymere und deren Mischungen umfasst.

31. Kaugummi-Granulat nach irgendeinem der Ansprüche 23 bis 30, in dem das Granulat im Wesentlichen frei von Schmiermitteln, Antihaftmitteln und Gleitmitteln ist.

## Revendications

1. Tablette de gomme à mâcher compressée (37) comprenant un centre de gomme à mâcher (38),
ledit centre de gomme (38) comprenant un comprimé de granulés de gomme de base (32) et des additifs de gomme à mâcher (33),
lesdits additifs de gomme à mâcher comprenant des édulcorants et des aromatisants,
au moins une première partie desdits granulés de gomme de base (32) comprenant une gomme de base à aromatisants ou ingrédients actifs incorporés,
au moins une seconde partie desdits granulés de gomme de base (32) comprenant des granulés de gomme de base conventionnelle.

2. Tablette de gomme à mâcher compressée selon la revendication 1, dans laquelle au moins ladite première partie desdits granulés de gomme de base (32) comprend une résine résistante aux aromatisants.

3. Tablette de gomme à mâcher compressée selon la revendication 1 ou 2, dans laquelle au moins ladite première partie desdits granulés de gomme de base (32) comprend une résine synthétique.

4. Tablette de gomme à mâcher compressée selon l'une quelconque des revendications 1 à 3, dans laquelle ladite résine synthétique comprend du poly(acétate de vinyle), des copolymères d'acétate de vinyle-laurate de vinyle et leurs mélanges.

5. Tablette de gomme à mâcher compressée selon l'une quelconque des revendications 1 à 4, dans laquelle au moins ladite première partie desdits granulés de gomme de base (32) est pratiquement exempte de cire.

6. Tablette de gomme à mâcher compressée selon l'une quelconque des revendications 1 à 5, dans laquelle ladite tablette de gomme à mâcher a une teneur en eau inférieure à 5 % en poids, de préférence inférieure à 3 % en poids, de manière encore plus préférable inférieure à 1 % en poids.

7. Tablette de gomme à mâcher compressée selon l'une quelconque des revendications 1 à 6, dans laquelle ledit centre de gomme (38) est pratiquement exempt d'adjuvants de compression.

8. Tablette de gomme à mâcher compressée selon l'une quelconque des revendications 1 à 7, dans laquelle au moins ladite seconde partie desdits granulés de base de gomme (32) a un caractère poisseux modéré.

9. Tablette de gomme à mâcher compressée selon l'une quelconque des revendications 1 à 8, dans laquelle au moins ladite seconde partie desdits granulés de gomme de base (32) comprend des résines naturelles.

10. Tablette de gomme à mâcher compressée selon l'une quelconque des revendications 1 à 9, dans laquelle au moins ladite seconde partie desdits granulés de gomme de base (32) comprend une cire.

11. Tablette de gomme à mâcher compressée selon l'une quelconque des revendications 1 à 10, dans laquelle ledit caractère poisseux modéré est obtenu au moyen d'au moins une résine naturelle incorporée dans au moins une partie des granulés de base de gomme (32).

12. Tablette de gomme à mâcher compressée selon l'une quelconque des revendications 1 à 11, dans laquelle la tablette de gomme à mâcher compressée comprend de 3 % à 50 % en poids de résines naturelles, de préférence de 5 % à 40 % en poids.

13. Tablette de gomme à mâcher compressée selon l'une quelconque des revendications 1 à 12, dans laquelle la tablette de gomme à mâcher compressée comprend de 0,5 % à 30 % en poids d'élastomères, de préférence de 5 % à 25 % en poids.

14. Tablette de gomme à mâcher compressée selon l'une quelconque des revendications 1 à 13, dans laquelle la tablette de gomme à mâcher compressée comprend de 0,1 % à 15 % en poids d'agents aromatisants, de préférence de 0,8 % à 5 % en poids.

15. Tablette de gomme à mâcher compressée selon l'une quelconque des revendications 1 à 14, dans laquelle les résines naturelles confèrent à la tablette une texture collante et améliorée.

16. Tablette de gomme à mâcher compressée selon l'une quelconque des revendications 1 à 15, dans laquelle ladite couche barrière comprend par exemple des lubrifiants, des anti-adhérents et des glissants.

17. Tablette de gomme à mâcher compressée selon l'une quelconque des revendications 1 à 16, dans laquelle la couche barrière comprend du stéarate de magnésium.

18. Tablette de gomme à mâcher compressée selon l'une quelconque des revendications 1 à 17, dans laquelle ladite couche barrière comprend des stéarates métalliques, des huiles végétales hydrogénées, des huiles végétales partiellement hydrogénées, des polyéthylène glycols, des monostéarates de poly(oxyéthylène), des graisses animales, des silicates, du dioxyde de silicates, du talc, des stéarates de magnésium, des stéarates de calcium, de la silice fumée, des huiles de graines de cotonnier hydrogénées en poudre, des huiles végétales hydrogénées, de l'huile de soja hydrogénée et leurs mélanges.

19. Tablette de gomme à mâcher compressée selon l'une quelconque des revendications 1 à 18, dans laquelle le centre de gomme est pratiquement exempt de lubrifiants, d'anti-adhérents et de glissants.

20. Gomme à mâcher compressée selon l'une quelconque des revendications 1 à 19, dans laquelle lesdits additifs de gomme à mâcher comprennent des édulcorants en une quantité comprise entre 5 et 95 % en poids de la gomme à mâcher, plus typiquement entre 20 et 80 % en poids, par exemple de 30 à 60 % en poids de la gomme.

21. Procédé de fabrication d'une gomme à mâcher compressée comprenant les étapes suivantes :
- incorporation d'au moins un élastomère et d'au moins un plastifiant dans une première base de gomme homogène,
- incorporation d'une quantité d'aromatisant dans ladite première base de gomme, de préférence par mélange mécanique,
- granulation de ladite base de gomme à aromatisant incorporé,
- incorporation d'au moins un élastomère et d'au moins un plastifiant dans une seconde base de gomme homogène,
- granulation de ladite seconde base de gomme,
- mélange de ladite première et de ladite seconde base de gomme durant l'addition d'autres additifs de gomme à mâcher et
- compression du mélange en une tablette.

22. Procédé selon la revendication 21, dans lequel la granulation est effectuée durant l'addition d'au moins un édulcorant en vrac.

23. Granulés de gomme à mâcher dans lesquels
une première partie des granulés de gomme à mâcher comprend des granulés dans lesquels ont été incorporés un aromatisant et/ou des ingrédients actifs
une seconde partie des granulés de gomme à mâcher comprend des granulés à base de gomme conventionnelle.

24. Granulés de gomme à mâcher selon la revendication 23, dans lequel la première partie des granulés de gomme à mâcher comprend des résines synthétiques.

25. Granulés de gomme à mâcher selon la revendication 23 ou 24, dans lequel la seconde partie des granulés de gomme à mâcher comprend une base de gomme fabriquée à partir de résines naturelles.

26. Granulés de gomme à mâcher selon l'une quelconque des revendications 23 à 25, dans lequel la première partie des granulés de gomme à mâcher est pratiquement exempte de résines naturelles.

27. Granulés de gomme à mâcher selon l'une quelconque des revendications 23 à 26, dans lequel la première partie des granulés de gomme à mâcher est pratiquement exempte de cire.

28. Granulés de gomme à mâcher selon l'une quelconque des revendications 23 à 27, dans lequel les granulés de gomme à mâcher comprennent un adjuvant de broyage, de préférence des édulcorants en vrac.

29. Granulés de gomme à mâcher selon la revendication 28, dans lequel ledit adjuvant de broyage comprend du sorbitol.

30. Granulés de gomme à mâcher selon l'une quelconque des revendications 23 à 29, dans lequel ladite résine synthétique comprend du poly(acétate de vinyle), des copolymères d'acétate de vinyle-laurate de vinyle et leurs mélanges.

31. Granulés de gomme à mâcher selon l'une quelconque des revendications 23 à 30, dans lequel les granulés sont pratiquement exempts de lubrifiants, d'anti-adhérents et de glissants.
